# EUROPEAN PATENT APPLICATION

(11) **EP 1 201 138 A1**
(43) Date of publication of application: **02.05.2002**
(21) Application number: 01125635.1
(22) Date of filing: 26.10.2001
(51) Int. Cl.: A23L 1/29, A23L 1/30, A23L 1/302, A23L 1/303, A23L 1/0524, A23L 1/0532, A23L 1/0562, A23L 1/22, A23L 1/275, A61K 9/14, A61K 9/16, A61K 9/50, A61K 9/51

(54) **Carotenoid beads**

(30) Priority: 27.10.2000 JP 2000328231
(71) Applicant: RIKEN VITAMIN CO., LTD., Chiyoda-ku, Tokyo (JP)
(72) Inventor: Sadano, Shin, Kawarajiri, Kawarabayashi, Kameoka Kyoto (JP); Sonoda, Tadamichi, Chiyoda-ku, Tokyo (JP)
(74) Representative: TER MEER STEINMEISTER & PARTNER GbR

(57) **Abstract**

The subject of the invention is to prepare carotenoid beads with high concentration and with less bleeding.

The subject can be accomplished by carotenoid beads which prepared by spraying dispersion added mixture of carotenoid to solution of gelatin, pectin or the like and medium chain fatty acid triglyceride comprised of fatty acid such as caprylic acid, capric acid or lauric acid.

## Description

### BACKGROUND OF THE INVENTION

The present invention relates to multicore type carotenoid beads . In detail, it relates to multicore type carotenoid beads prepared by using mixture of water-insoluble carotenoid crystal and medium chain fatty acid triglyceride (MCT) as a core substance.

Carotenoid is a compound characterized by having many conjugated double bond. It is a generic term of pigment contained in animal fat of plants such as β-carotene, α-carotene, lutein, zeaxanthin, capsanthin, lycopene, β-cryptoxanthin, canthaxanthin and astaxanthin, and has been used since long ago as a yellow to red pigment.

Moreover, it has been known since long ago that (3-carotene has provitamin A activity. In recent years, not only the provitamin A effect of β-carotene was recognized, but also the antioxidative action in vivo was recognized for whole carotenoid, and further many physiological activities have been recognized in addition to the anticancer and antitumor effects. Therefore, recently it has been noticed as the health food and nutrition supplementary agent.

Most of these carotenoids are insoluble in water and have low solubility to oil and fat. Furthermore, since they have properties very liable to be oxidized, they are produced usually in the state dispersed in vegetable oil for preventing oxidation.

On the other hand, when utilizing the carotenoid for the health food and nutrition supplementary food, it is used together with vitamin, mineral, etc. for making a tablet type supplement. As above-mentioned, since the carotenoid is liable to be oxidized in the state of crystal, when adding it to supplement as it is, it deteriorates for a very short time. Moreover, in the state of being dissolved in vegetable oil, good supplement tablet cannot be obtained.

Therefore, it is used in the shape of multicore type hard beads obtained by granulating an emulsion/dispersion of carotenoid-containing vegetable oil in aqueous solution of film-forming substance containing gelatin without air permeability as a major component into small granules, and then by drying. Depending on the components in supplement, they dislike to be mixed each other in some cases, and it is necessary for beads to withstand the tablet-making pressure and not to cause the destruction of granule or the bleeding of oil component. The carotenoid used for these hard beads of multicore type is used by dispersing in liquid vegetable oils such as corn oil, rapeseed oil and soybean oil as described above, and the beads are obtained by emulsifying /dispersing this in aqueous solution of film-forming substance containing gelatin as a major component, then granulating and drying.

However, the vegetable oil composition, in which carotenoid is dispersed, has high viscosity, hence it is not easy to emulsify/disperse this in aqueous solution containing gelatin as a major component for preparation of hard beads, keeping the size of oil droplets homogeneous and in a stable state, resulting in that the size of oil droplets inside of dried beads is liable to become uneven. When making the tablet of supplement using beads in such state, the bleeding of oil droplets from portions of coarse oil droplets in beads occurs by the pressure applied on tablet-making, leading to lower quality of supplement as a commodity by causing uneven color or stripes of color on the surface of tablet.

### SUMMARY OF THE INVENTION

The subject of this invention is to prepare multicore carotenoid beads with less bleeding of carotenoid onto the surface of beads on tablet-making.

As a result of diligent studies to solve said subject, the inventors have found that the subject is cleared up by using a solution or dispersion of carotenoid in medium chain fatty acid triglyceride as core substance, leading to the completion of the invention.

### DETAILED DESCRIPTION OF THE INVENTION

The medium chain fatty acid triglyceride to be used in this invention is a triglyceride constituted one or more kinds of fatty acids with number of carbon atoms of 6 to 12 such as caproic acid, caprylic acid, capric acid or lauric acid. Preferably the fatty acid constituted of triglyceride is a mixture of caprylic acid and capric acid and it is liquid at ambient temperature.

The carotenoids on this invention are water-insoluble carotenoids such as β-carotene, α-carotene, lutein, zeaxanthin, capsanthin, lycopene, β-cryptoxanthin, astaxanthin and canthaxanthin.

For the film-forming substances to be used in this invention, gelling agents for food use such as gelatin, pectin, casein, alginic acid, ferlacene, agar and jellan gum are used as major components, and polyhydric alcohols such as glycerine, trehalose, sucrose and sorbit, etc. can be formulated to adjust the physical properties.

For preparing the beads of this invention, fine carotenoid crystal is dissolved or dispersed in medium chain fatty acid triglyceride and, on the other side, aqueous solution of well-known water-soluble high-molecular substance such as gelatin or pectin is produced as a film-forming substance for the preparation of beads, then both are mixed and the mixture of carotenoid/medium chain fatty acid triglyceride is emulsified/dispersed in the aqueous solution of high-molecular substance by using high-speed homogenizer. This dispersion is atomized into cool atmosphere or dispersed into water-insoluble liquid such as vegetable oil through extrusion nozzles to coagulate the water-soluble high-molecular substance solution into the shape of particles. When coagulating in a solvent of water-insoluble liquid, the solvent is removed by filtration etc. and further, the solvent adhered to the surface of coagulates is removed by washing with low-boiling point solvent such as ethanol, in which the water-soluble high-molecular substance does not dissolve, then the coagulates are dried to obtain the multicore type beads being the object of the invention.

In this invention, except that the oil used for dissolving or dispersing the carotenoid is replaced from vegetable oil used hitherto to medium chain fatty acid triglyceride, traditional publicly known method can be used, hence the manufacturing method is not particularly restricted.

In following, the invention will be illustrated based on the examples.

### <Example 1>

60 grams of purified lutein, 2.7g of mix tocopherol (Riken E oil 700, from Riken Vitamin Co.) and 28.2g of medium chain fatty acid triglyceride (Actor M-1, from Riken Vitamin Co.) were warmed at 78°C, and lutein was dispersed homogeneously under stirring, using TK homomixer (from Tokushu Kika Kogyo Co.). Dispersion was added into a solution of 80°C containing 120g of gelatin, 60g of granulated sugar and 450g of water mixed, warmed and dissolved beforehand, and the mixture was dispersed homogeneously using TK homomixer. The pasty liquid obtained was atomized onto a bed of starch powder under cold condition of about 10°C by using centrifugal nozzles. The granules were dried by fluidized dryer for 2 hours at 30°C and subsequently for 3 hours at 50°C, and then starch was removed by sieving to obtain lutein beads.

### <Example 2>

Lutein in Example 1 was replaced with astaxanthin, then astax anthin beads were obtained by treatment similarly to Example 1.

### <Example 3>

Lutein in Example 1 was replaced with β-carotene, then β-carotene beads were obtained by treatment similarly to Example 1.

### [Comparative example]

Medium chain fatty acid triglyceride in Example 1 was replaced with rapeseed oil, lutein beads were obtained by treatment similarly to Example 1.

### [Preservation test in vessel]

The beads obtained in Examples 1 to 3 and Comparative example were taken on filter paper in petri dish, and kept in 30°C incubator, then the coloring state of filter paper caused by bleeding of carotenoid was observed. While coloring was hardly perceived on each sample of Example 1 to 3, the filter paper of Comparative example colored to yellow, hence dissolving-out of lutein from beads was perceived.

### [Tablet-making test]

With respective beads obtained in Examples 1 to 3 and Comparative example, 200mg of bead, 1, 800mg of sorbitol granule and 20mg of sucrose fatty acid ester (DK ester S-20W, from Dai-ichi Kogyo Seiyaku Co.) were mixed and put into a mortar with diameter of 18mm. Mixture was pressed under load of 3 tons per one tablet, and then the bleeding state of carotenoid to each tablet was observed by external appearance.

With the samples of Examples 1 to 3, little bleeding was perceived, and, with the sample of Comparative example, yellow specks or stripe-like bleeding were observed.

In accordance with the method of this invention, beads with less dissolving-out of carotenoid can be obtained, thus allowing to apply preferably to tablet type supplement and to give tablet with excellent quality.

## Claims

1. Multicore type carotenoid beads comprising of mixture being dissolved or dispersed carotenoid in medium chain fatty acid triglyceride as core substances.

2. The multicore type carotenoid beads defined in claim 1, wherein the medium chain fatty acid triglyceride is constituted by one or more kind of fatty acid selected from fatty acids with number of carbon atoms of 6 to 12.

3. A method of manufacturing multicore type carotenoid beads comprising the steps of dissolving or dispersing carotenoid in a medium chain fatty acid triglyceride as core substances, adding and dispersing this solution or dispersion to a solution of film-forming substance, and then converting the obtained dispersion to beads.
